# EUROPEAN PATENT APPLICATION

(11) **EP 2 479 319 A1**
(43) Date of publication of application: **25.07.2012**
(21) Application number: 10815301.6
(22) Date of filing: 01.09.2010
(51) Int. Cl.: C30B 29/58, C07K 1/14, C30B 7/00, C30B 30/00

(54) **CONTAINER FOR CRYSTALLIZATION, CRYSTALLIZATION APPARATUS, METHOD FOR PRODUCING CRYSTAL, AND SUBSTRATE FOR CRYSTALLIZATION**

(30) Priority: 14.09.2009 JP 2009211912
(71) Applicant: National University Corporation Gunma University, Maebashi-shi, Gunma 371-8510 (JP); National University Corporation Hokkaido University, Sapporo-shi, Hokkaido 060-0808 (JP)
(72) Inventor: The designation of the inventor has not yet been filed
(74) Representative: Vossius & Partner
(86) International application number: PCT/JP2010/064908
(87) International publication number: WO 2011/030704

(57) **Abstract**

A container for crystallization of a biopolymer of the invention is provided that includes a structure wherein two or more noble metals and/or noble metal-coated bodies are arranged at an interval of 1 to 1,000 nm. There are also provided a crystallization apparatus of a biopolymer, comprising the container for crystallization of a biopolymer, a method for producing a biopolymer crystal, comprising the steps of preparing the container for crystallization of a biopolymer, and making the structure contact with a biopolymer solution, and a substrate for crystallization of a biopolymer, having a structure wherein two or more noble metals and/or noble metal-coated bodies are arranged at an interval of 1 to 1,000 nm.

## Description

The present invention relates to a container for crystallization, a crystallization apparatus, a method for producing a crystal, and a substrate for crystallization, and to a container for crystallization, a crystallization apparatus, a method for producing a crystal, and a substrate for crystallization that can be applied particularly preferably when the crystal is a biopolymer crystal.

In order to crystallize biopolymers such as protein, such methods as a batch method, a dialyzing method, a liquid-liquid phase diffusion method and a gas-liquid phase diffusion method are used (see Non-patent Document 1).

For example, according to the batch method, to a protein solution, a precipitation agent such as ammonium sulfate is added directly up to a crystallization concentration. Generally, to a container that is charged with a biopolymer solution, the precipitation agent is added and controlled so that the biopolymer is led to supersaturation, to fabricate a biopolymer crystal. In the batch method, there are such defects that a polymer sample of a high concentration is required in a large quantity, that the operation requires an experience and skill and shows a low reproducibility, and that screening of crystallization conditions is difficult. And, conventional crystallization methods described above have such a defect that they require comparatively peculiar crystallization conditions for specific polymers and there is no general-purpose condition.

For example, Patent Document 1 discloses a technique wherein laser light that is considered to be in a visible region is irradiated to a protein solution that is set at a temperature condition suitable for nucleus formation of a protein crystal, a scattering situation of the laser light is analyzed to detect the beginning of the nucleus formation of the protein crystal, and the protein solution is controlled so as to be a temperature condition suitable for the crystal growth, at the time when the beginning of the nucleus formation is detected.

Patent Document 2 discloses a method of making a crystal of hen egg white lysozyme appear by irradiating fourth harmonic 266 nm light of a neodymium YAG laser, or 500 W xenon lamp light to a solution (a metastable solution) that is in a supersaturated state but is at a low supersaturation degree that is unsuitable for the nucleus formation of a protein crystal.

Patent Document 3 discloses a method for producing a crystalline nucleus wherein a crystalline nucleus is grown by irradiating at least one of pulse lasers of a pico-second pulse laser and a femto-second pulse laser to a solution dissolving a solute being a compound of the crystallization.
Patent Document 1: JP-A-6-116098 (JP-A denotes a Japanese unexamined patent application publication)
Patent Document 2: JP-A-2003-306497
Patent Document 3: WO 2004/018744

Non-patent Document 1: Chapter 14 "Crystallization" written by Tsunehiro Takano; New Biochemical Experimental Course 1 "Protein I - Separation, Purification, Nature -" edited by The Japanese Biochemical Society, published by TOKYO KAGAKU DOZIN CO., LTD., 1990

A purpose of the invention is to provide a container for crystallization, a crystallization apparatus, a method for producing a crystal, and a substrate for crystallization capable of producing simply and effectively a crystal, preferably a biopolymer crystal.

The above-described problems were solved by means for solving the problems described in <1>, <7>, <8> or <11> below. With <2> to <6>, <9> and <10> that are preferable embodiments, they are described below.
<1> A container for crystallization of a biopolymer, comprising a structure wherein two or more noble metals and/or noble metal-coated bodies are arranged at an interval of 1 to 1,000 nm,
<2> the container for crystallization of a biopolymer according to <1> above, wherein the noble metal is gold, silver, platinum, and/or an alloy thereof,
<3> the container for crystallization of a biopolymer according to <1> or <2> above, wherein the noble metal is gold,
<4> the container for crystallization of a biopolymer according to any one of <1> to <3> above, wherein at least two among the noble metals and/or the noble metal-coated bodies are arranged at an interval of 1 to 500 nm,
<5> the container for crystallization of a biopolymer according to any one of <1> to <4> above, wherein the container has the structure that is directly provided on the surface of the container, or the container has a substrate for which the structure is provided, in the container,
<6> the container for crystallization of a biopolymer according to any one of <1> to <5> above, wherein the length, the width, and the height of the noble metals and/or noble metal-coated bodies is each independently 5 to 500 nm,
<7> a crystallization apparatus of a biopolymer, comprising the container for crystallization of a biopolymer according to any one of <1> to <6> above,
<8> a method for producing a biopolymer crystal, comprising the steps of preparing the container for crystallization of a biopolymer according to any one of <1> to <6> above, and making the structure contact with a biopolymer solution,
<9> the method for producing a biopolymer crystal according to <8> above, further comprising a step of irradiating light to the structure contacting with the biopolymer solution,
<10> the method for producing a biopolymer crystal according to <9> above, wherein the light is a light having a wavelength that is longer than 400 nm, and
<11> a substrate for crystallization of a biopolymer, having at least a structure wherein two or more noble metals and/or noble metal-coated bodies are arranged at an interval of 1 to 1,000 nm.

According to the present invention, it was possible to provide a container for crystallization, a crystallization apparatus, a method for producing a crystal, and a substrate for crystallization that were capable of producing simply and effectively a crystal, preferably a biopolymer crystal.

### Brief Description of the Drawings

Fig. 1 is an upper surface schematic view showing an example of the container for crystallization of the present invention, and an example of the substrate for crystallization of the invention.
Fig. 2 is an upper surface schematic view showing an example of the structure wherein two or more noble metals and/or noble metal-coated bodies are arranged at an interval of 1 to 1,000 nm in the invention.
Fig. 3 is an upper surface schematic view showing another example of the structure wherein two or more noble metals and/or noble metal-coated bodies are arranged at an interval of 1 to 1,000 nm in the invention.
Fig. 4 is a drawing showing the result of Example 1.
Fig. 5 is a partially enlarged drawing wherein a part of Fig. 4 is enlarged.
Fig. 6 is a drawing showing the result of Comparative Example 1.
Fig. 7 is a drawing showing the result of Comparative Example 2.
Fig. 8 is a drawing showing the result of Example 2.
Fig. 9 is a partially enlarged drawing wherein a part of Fig. 8 is enlarged.
Fig. 10 is a drawing showing the result of Comparative Example 3.
Fig. 11 is a drawing showing the result of Comparative Example 4.

### Description of Reference Numerals and Signs

10: substrate for crystallization, 12: noble metal nano structure, 14: substrate, 16: noble metal

Hereinafter, the present invention is described in detail.

### (The container for crystallization, and the substrate for crystallization)

The container for crystallization of the invention is characterized by having the structure wherein two or more noble metals and/or noble metal-coated bodies are arranged at an interval of 1 to 1,000 nm.

The container for crystallization of the invention can be used preferably as a container for crystallization of a biopolymer.

The substrate for crystallization of the invention is characterized by having the structure wherein two or more noble metals and/or noble metal-coated bodies are arranged at an interval of 1 to 1,000 nm.

The substrate for crystallization of the invention can be used preferably as a substrate for crystallization of a biopolymer.

As the result of having the structure wherein two or more noble metals and/or noble metal-coated bodies are arranged at an interval of 1 to 1,000 nm, the container for crystallization of the invention and the substrate for crystallization of the invention can produce simply and effectively a crystal, preferably a biopolymer crystal.

Compounds to be crystallized using the container for crystallization of the invention and the substrate for crystallization of the invention are not particularly limited, but may be inorganic compounds, organic compounds or high molecular compounds, and preferably are biopolymers. For example, when the container for crystallization of the invention and/or the substrate for crystallization of the invention is used for the crystallization of a biopolymer, if a strong laser light or ultraviolet light is irradiated to a solution of the biopolymer, as is the methods described in Patent Documents 1 to 3, the biopolymer may be denaturalized, and, therefore, it is preferable to induce the crystallization without irradiating a strong laser light or ultraviolet light.

Specific examples of the biopolymers can include polypeptides, proteins, nucleic acids (for example, such as DNA), and derivatives thereof, etc. The biopolymer includes synthesized materials such as synthesized polypeptides and synthesized proteins. The polypeptide includes polypeptides isolated by a common method after being obtained by the expression in E. coli, yeasts, or animal cells, and synthesized polypeptides. The derivatives include, for example, glycoproteins, DNA conjugates, etc.

The (weight average) molecular weight of the biopolymer is preferably not less than 1,000, more preferably from not less than 1,000 to not more than 1,000,000.

Among these, as the biopolymer, a polypeptide, a protein and a derivative thereof are preferable, and a protein and a derivative thereof (in the invention, also called simply "the protein") are more preferable. The protein includes an enzyme.

The container for crystallization of the invention may have the structure wherein two or more noble metals and/or noble metal-coated bodies are arranged at an interval of 1 to 1,000 nm (hereinafter, also called simply "the noble metal nano structure") that is provided directly on the container surface, or may have a structural body that is provided with the structure in the container. When the container for crystallization of the invention has the structural body, the container itself may be bound physically or chemically to the structural body, or may not. Specifically, for example, the structural body may be adhered in the container for crystallization of the invention, or the structural body may simply be placed in the container.

The structure to be provided for the container for crystallization of the invention, or for the substrate for crystallization of the invention may be formed by the noble metal itself, or may be formed by a noble metal-coated body, but the formation by the noble metal itself is preferable. That is, the container for crystallization of the invention, or the substrate for crystallization of the invention at least has, preferably, the structure wherein two or more noble metals are arranged at an interval of 1 to 1,000 nm.

As the noble metals in the structure wherein two or more noble metals and/or noble metal-coated bodies are arranged at an interval of 1 to 1,000 nm, gold, silver, platinum, and/or alloys thereof are preferable, gold, silver or platinum is more preferable, and gold is particularly preferable.

In the noble metal-coated body, a part thereof may be coated with the noble metal, and surfaces other than the surface contacting with the container or the substrate for which the noble metal-coated body is provided, preferably, are coated with the noble metal. No particular limitation is imposed on the inside of the noble metal-coated body, but the inside may be metal, glass, a noble metal of a kind other than the noble metal for the coating.

In the container for crystallization of the invention, or the substrate for crystallization of the invention, regarding the noble metals and/or noble metal-coated bodies, at least two are arranged at an interval of 1 to 1,000 nm, preferably at least two are arranged at an interval of 1 to 750 nm, more preferably at least two are arranged at an interval of 1 to 500 nm, and yet more preferably at least two are arranged at an interval of 1 to 300 nm.

The shape of the noble metals and/or the noble metal-coated bodies is not particularly limited, and the shape may be arbitrary. Examples thereof include a polygonal prism shape, a cylinder shape, a polygonal pyramid shape, a cone shape, a granular shape and irregular shapes etc., and the shape is preferably a polygonal prism shape, a cylinder shape or a granular shape, more preferably a polygonal pyramid shape, and yet more preferably a cubic shape or a cuboid shape.

The height of the noble metals and/or alloys thereof (the height from the container or the substrate for which they are provided) is preferably 1 to 1,000 nm, more preferably 5 to 500 nm, and yet more preferably 10 to 100 nm.

A circle-equivalent diameter (diameter) obtained from a projected area of the noble metals and/or the noble metal-coated bodies, which is projected from a direction vertical to the surface of the container or the substrate for which the structure is provided, is preferably 1 to 500 nm, more preferably 10 to 300 nm, and yet more preferably 20 to 200 nm.

The longest lengths of the length and the width of the noble metals and/or the noble metal-coated bodies (the length and the width in the direction parallel to the surface of the container or the substrate for which they are provided) each independently is preferably 1 to 1,000 nm, more preferably 10 to 500 nm, and yet more preferably 50 to 300 nm.

The noble metals and/or the noble metal-coated bodies in the noble metal nano structure is not particularly limited as long as at least two thereof are arranged at an interval of from 1 to 1,000 nm, but they are formed preferably in a region of at least 0.1 mm × 0.1 mm over the substrate, more preferably in a region of at least 0.5 mm × 0.5 mm over the substrate, and yet more preferably in a region of at least 1 mm × 1 mm over the substrate.

The number of the noble metals and/or the noble metal-coated bodies in the noble metal nano structure is 2 or more, preferably 4 to 100,000, and more preferably 9 to 10,000.

The container for crystallization of the invention, or the substrate for crystallization of the invention may have only one noble metal nano structure, or may have 2 or more noble metal nano structures.

The shape of the container for crystallization of the invention is not particularly limited as long as the container has a shape capable of making the noble metal nano structure contact with a solution containing a material to be crystallized, and any desired shape is acceptable. Also, the shape of the substrate for crystallization of the invention is not particularly limited, the shape may be planer or not planar, and any desired shape is acceptable.

The container for crystallization of the invention is preferably a sealable container in order to suppress evaporation of a solution containing a compound to be crystallized in the crystallization.

The substrate for forming the noble metal nano structure in the substrate for crystallization of the invention is not particularly limited, but a transparent substrate is preferable from the viewpoint of performing easily the check of crystal generation and the light irradiation, and a glass substrate is more preferable.

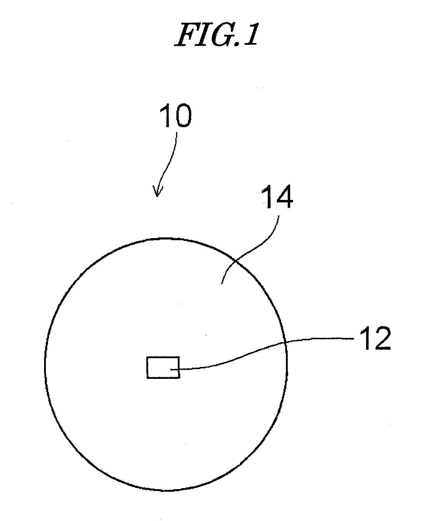

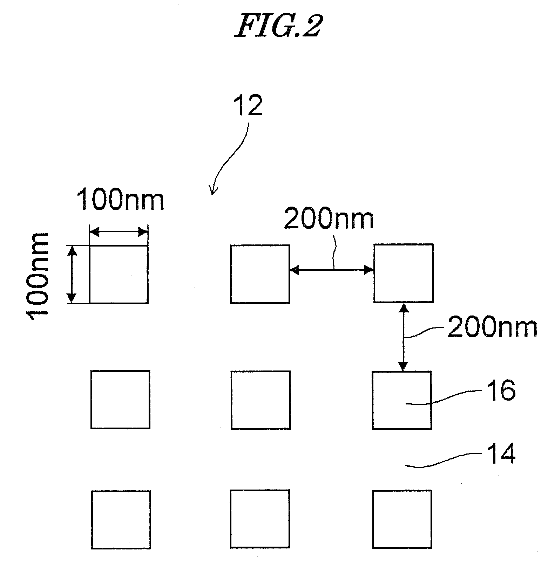

Preferably specific examples of the container for crystallization of the invention and the substrate for crystallization of the invention include those shown in Figs. 1 and 2.

Fig. 1 is an upper surface schematic view showing an example of the substrate for crystallization of the invention, and Fig. 2 is an upper surface schematic view showing an example of the structure wherein two or more noble metals and/or noble metal-coated bodies are arranged at an interval of 1 to 1,000 nm in the substrate for crystallization in Fig. 1.

A substrate for crystallization 10 shown in Fig. 1 is one wherein a structure (a noble metal nano structure) 12, in which two or more noble metals are arranged at an interval of 1 to 1,000 nm, is formed in a region of 0.5 mm × 1.0 mm at the central part over a circular substrate 14.

Fig. 2 is an upper surface schematic view wherein a part of the noble metal nano structure 12 formed in Fig. 1 is enlarged, and, in the noble metal nano structure 12 shown in Fig. 2, a noble metal 16 is formed in a cuboid shape of 100 nm × 100 nm × height 30 nm (not shown), and the respective noble metals are formed at an interval of 200 nm longitudinally and horizontally, respectively, over the substrate 14.

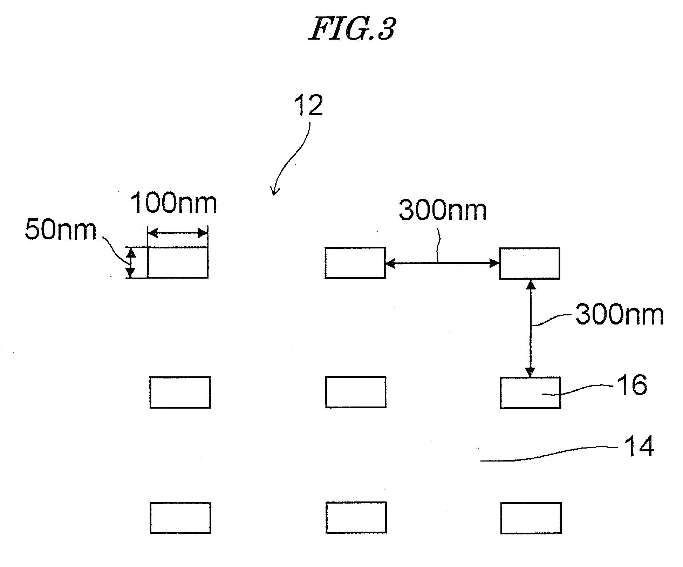

Fig. 3 is an upper surface schematic view showing another example of the structure wherein two or more noble metals and/or noble metal-coated bodies are arranged at an interval of 1 to 1,000 nm in the invention.

In the noble metal nano structure 12 shown in Fig. 3, the noble metal 16 is formed in a cuboid shape of 100 nm × 50 nm × height 40 nm (not shown), and the respective noble metals are formed at an interval of 300 nm longitudinally and horizontally, respectively, over the substrate 14.

### (Method for producing a crystal)

The method for producing a crystal of the invention comprises the steps of preparing the container for crystallization of the invention (hereinafter, also called a "preparation step"), and making the structure contact with a solution of a material to be crystallized (hereinafter, also called a "contacting step").

The method for producing a crystal of the invention can preferably be used as a method for producing a biopolymer crystal. The solution of a material to be crystallized is preferably a biopolymer solution.

It is preferable that the method for producing a crystal of the invention further comprises a step of irradiating light to the structure contacting with the solution of a material to be crystallized (hereinafter, also called a "light irradiation step"), from the viewpoint of more accelerating the crystal generation.

And, It is preferable that the method for producing a crystal of the invention further comprises a step of storing the solution of a material to be crystallized (hereinafter, also called a "storing step") after the contacting step or the light irradiation step.

### <Preparation step>

The method for producing a crystal of the invention comprises a step of preparing the container for crystallization of the invention (a preparation step).

The container for crystallization of the invention in the preparation step may be provided with the noble metal nano structure directly on the container surface, or may have a structural body provided with the structure in the container.

A method for forming the noble metal nano structure in the container for crystallization of the invention or the substrate for crystallization of the invention is not particularly limited, and a known method can be used. For example, it can be formed by a known semiconductor fine processing technique. Specifically examples thereof include a method wherein resist is coated on the surface of the substrate, for the resist, the shape of an intended noble metal nano structure is drawn with electron beams, the drawn image is developed to expose the substrate in accordance with the shape of the noble metal nano structure, a noble metal is sputtered from above the developed surface to form a noble metal film, and an unnecessary metal film is removed with the resist.

As the resist, a known one can be used. The resist preferably has a thickness of 200 nm or less. In order to make the thickness thin, preferably the concentration of the resist solution to be coated is lowered. An acceleration voltage of electron beams in the drawing is preferably 100 to 200 kV. No particular limitation is imposed on the dose rate of the exposure, but the rate may appropriately be selected. Developing time for removing the drawn resist may be a time capable of removing sufficiently the resist, and, for example, the time may appropriately be determined in accordance with the above-mentioned respective conditions.

The method for forming the noble metal nano structure may also refer to JP-A-2007-71667, JP-A-2008-6575, etc.

### <Contacting step>

The method for producing a crystal of the invention comprises the step of making the structure wherein two or more noble metals and/or noble metal-coated bodies are arranged at an interval of 1 to 1,000 nm in the container for crystallization of the invention contact with a solution of a material to be crystallized (a contacting step).

In the contacting step, no particular limitation exists, except for making the structure contact with the solution of a material to be crystallized.

The "solution of a material to be crystallized" may be a liquid containing a material to be crystallized and a solvent capable of dissolving the material to be crystallized, wherein the liquid is preferably a solution that has dissolved completely the material to be crystallized.

The solvent for use in the solution of a material to be crystallized can independently be selected in accordance with respective materials to be crystallized to be used, and includes, for example, water, an organic solvent, a mixture of water and a water-miscible organic solvent (an aqueous organic solvent) etc.

Among these, when a biopolymer is used as the material to be crystallized, a buffer solution is preferable, and an acetic acid buffer solution, a CAPS buffer solution, a HEPES buffer solution, a citric acid buffer solution, a tartaric acid buffer solution, a cacodylic acid buffer solution, or a Tris buffer solution is more preferable. In the case of a biopolymer of an amphoteric electrolyte, it is preferable to irradiate light to a biopolymer solution wherein pH has been adjusted near the isoelectric point thereof, and it is also preferable to prepare the mixed liquid having the adjusted pH.

The concentration of the material to be crystallized in the solution of a material to be crystallized is not particularly limited, and examples of the solution include a solution of 1 to 100% of the saturated concentration, and a supersaturated solution. The concentration is preferably 80% or more of the saturated concentration, more preferably 90% or more of the saturated concentration, and particularly preferably the saturated or supersaturated concentration.

In order to keep the solution concentration, replenishment of the material to be crystallized that is a solute, lowering of temperature, and/or addition of the precipitation agent etc. may be performed.

The solution of a material to be crystallized in the invention may contain a crystallization agent.

The "crystallization agent" in the invention means a compound that acts to lower the solubility of the material to be crystallized, preferably the biopolymer, and such compounds are mentioned as the precipitation agent, a pH buffering agent, other additives that are used for crystallization of a polymer, etc.

Examples of the crystallization agents that are usable in the invention include salts, organic solvents, water-soluble polymers etc., wherein a known one can be used. A kind of the crystallization agent to be used may appropriately selected in accordance with the material to be crystallized to be used.

As the salts, sulfates, nitrates, phosphates, organic acid salts, and halides of alkali metals or alkaline earth metals can be used, and specifically examples thereof include ammonium sulfate, sodium chloride and sodium citrate.

As the organic solvent, water-soluble organic solvents can be exemplified. Specifically examples of the organic solvent that can be used include 2-methyl-2,4-pentadiol (MPD), ethanol, propanol, dioxane etc.

As the water-soluble polymers, polyethylene glycol, polypropylene glycol, etc. can be exemplified.

The addition amount of the crystallization agent is not particularly limited, but may appropriately be set in accordance with the material to be crystallized to be used and the kind of the crystallization agent to be used.

Biopolymers that can be used in the invention preferably has high purity and high homogeneity, because the crystal can be made more easily. Therefore, the method for producing a biopolymer crystal of the invention preferably comprises the step of purifying the biopolymer, prior to the production of the crystal.

The purification of biopolymers prior to the crystallization can be performed by a known method, and, for example, the purification is preferably performed by affinity chromatography, chromatography in common use, rpHPLC, FPLC, etc.

In a case where the crystal of a nucleic acid is to be produced, after isolation by a known isolation method, preferably the nucleic acid is purified to enhance the purity and then is crystallized.

In a case of protein, it is preferable to enhance the purity by a known method, to check the purity by isoelectric electrophoresis, a light scattering method or the like, and, after that, to perform the crystallization.

In addition to the material to be crystallized, the solvent and the crystallization agent, if necessary, a known additive may be added to the solution of a material to be crystallized. But, needless to say, it is necessary to consider so that no influence is given to the crystallization, in the storing step.

The addition may be performed once, or may be performed divided into a plurality of times.

### <Light irradiation step>

It is preferable that the method for producing a crystal of the invention further comprises a step of irradiating light to the structure, wherein two or more noble metals and/or noble metal-coated bodies are arranged at an interval of 1 to 1,000 nm, contacting with the solution of a material to be crystallized (a light irradiation step).

The "light" in the invention may be an electromagnetic wave such as ultraviolet light, visible light and infrared light.

The wavelength of light that is irradiated in the light irradiation step is not particularly limited, but the wavelength is preferably 400 nm or longer, more preferably 450 to 2,000 nm, yet more preferably 500 to 1,500 nm, and particularly preferably 500 to 1,200 nm.

The light to be irradiated in the light irradiation step includes preferably at least visible light and/or near-infrared light, and is more preferably only visible light and/or near-infrared light.

The visible light in the invention has preferably a wavelength of 400 to 780 nm.

The near-infrared light in the invention has preferably a wavelength of more than 780 nm to not more than 2,500 nm, more preferably a wavelength of more than 780 nm to not more than 2,000 nm.

The light to be irradiated in the light irradiation step may be monochromatic light or continuous light.

Intensity of the light to be irradiated may appropriately selected, but, usually, a light of an intensity in the range of several µW to several hundred W can be used.

The light irradiation may be performed by a fixed light or by a pulsed light. If necessary, irradiation intensity, energy per one pulse, pulse interval etc. may also be changed.

In the light irradiation, preferably a fixed light is continuously irradiated, but may be irradiated intermittently or may be interrupted on the way.

The light irradiation time in the light irradiation step is not particularly limited, but the light may be irradiated continuously or intermittently until the generation of the crystal.

### <Still-standing step>

It is preferable that the method for producing a crystal of the invention further comprises a step of storing the solution of a material to be crystallized (a storing step), after the contacting step or the light irradiation step.

Moreover, the storing step may be performed at the same time as the light irradiation step. For example, while performing the light irradiation, the solution of a material to be crystallized may be still-stood to generate the crystal.

The storing time may appropriately be selected under such conditions that enables growth of the material to be crystallized to be performed sufficiently, and may appropriately be determined in consideration of, for example, the material to be crystallized, the crystallization agent, a kind of the solvent used, presence or absence of crystal generation, size of a generated crystal, etc.

The temperature in the storing is not particularly limited as long as it is not a temperature that hinders the crystallization of the material to be crystallized. The temperature in the storing may be kept constant or be changed, but preferably the temperature change is preferably less than 1°C.

The solution of a material to be crystallized in the storing step may be placed and stored in a sealed container, or may be placed and stored in a unsealed container. In the inside and outside of the container, a solvent quantity in an atmosphere, for example, humidity may appropriately be set according to need. An atmosphere inside and outside the container may appropriately be selected in accordance with a kind of the material to be crystallized to be used, and, for example, may be air, a nitrogen atmosphere, or an argon atmosphere.

In the storing step, still-standing storage and storage with stirring are allowable, or, continuous, intermittent or temporary vibration may be given. The storage with stirring and/or vibration occasionally leads to give a large crystal.

A vibration number in the stirring in the storing step is preferably not less than 10 rpm and not more than 300 rpm, more preferably not less than 20 rpm and not more than 100 rpm, yet more preferably not less than 30 rpm and not more than 60 rpm.

### <Determination step>

It is preferable that the method for producing a crystal of the invention further comprises a step of determining the presence or absence of a crystal that might have been generated in the solution.

A method for determining the presence or absence of the crystal in the determination step is not particularly limited, but a method of visual observation, or a method that uses a sensor using an image processing or an optical technique is preferably exemplified.

The determination step is preferably performed periodically, if necessary, and, for example, the determination step may be performed after the start of the storing, after 1 day, after 2 days, after 3 days, after 5 days, after 7 days, after 30 days, after 60 days, and after 90 days, respectively.

When no crystal is generated after the check of the presence or absence of the crystal, the light irradiation step may further be performed for the solution wherein no crystal has been generated. If necessary, the light irradiation step and the storing step may be repeated many times until the generation of the crystal.

In the method for producing a crystal of the invention, the crystal that has been generated in the solution may be separated by an arbitrary method. Specifically, a method of filtration using filter paper, a filter or the like, a method of collecting the crystal with tweezers etc., and the like can be exemplified.

The obtained crystal may be subjected, if necessary, to washing, drying, processing of the size and shape, or recrystallization.

The system of crystallization in the method for producing a crystal of the invention is not particularly limited, but a known method can be used. In particular, when the crystallization of the biopolymer is performed, for example, such methods as a hanging drop vapor diffusion method, a sitting drop vapor diffusion method, a micro dialysis, a free interface diffusion method, a storage batch method, etc. can preferably be used.

Regarding other conditions for accelerating the crystallization of biopolymers, the above-cited Chapter 14 "Crystallization" written by Tsunehiro Takano; New Biochemical Experimental Course 1 "Protein I - Separation, Purification, Nature -" edited by The Japanese Biochemical Society, published by TOKYO KAGAKU DOZIN CO., LTD., 1990, and A. McPherson, "Preparation and Analysis of Protein Crystals" (John Wiley & Son, Inc.) can be referred to.

An apparatus usable for the method for producing a crystal of the invention is not particularly limited. A known method and apparatus may be combined.

The apparatus usable for the method for producing a crystal of the invention is preferably equipped with a means for irradiating light and a storage means, and, if necessary, may be equipped with various kinds of means such as a solvent-preparing means, a temperature-regulating means, a humidity-regulating means, a stirring means, a vibration means, a means for determining the presence or absence of the crystal, and a means for adding an additive.

In the method for producing a crystal of the invention, two or more apparatuses having one or more necessary means may be used in combination, or a single apparatus equipped with all the necessary means may be used.

As the vibration means, a known means of vibration, stirring, ultrasonic wave generation, etc. can be used.

As an oscillator in the vibration means, those of various configurations such as a piezoelectric oscillator, suction force or electromagnetic force may be mentioned, and no particular limitation is imposed only if it can give vibration.

As a method for giving vibration to the biopolymer solution, for example, there are mentioned a method wherein a container containing a solution dissolving a biopolymer is made to contact with a vibration means that is vibrating, a method wherein a container containing a solution dissolving a biopolymer is fixed to a plate and the whole plate is made to vibrate, etc.

The light irradiation means can be constituted, for example, from a light source, and an optical system for guiding the light to the solution. As optical parts such as a lens and a mirror that are used in the light path for guiding the light from the light source to the sample to be irradiated, the use of those that allow the light to pass through or to be reflected, effectively, is preferable.

As the light source, the above-mentioned light source of constant lighting or a laser light can be preferably used.

For the optical system, suitably, optical members such as a reflecting mirror, a condenser lens, a light filter, an infrared ray cutoff filter, an optical fiber, a light guide plate, and a nonlinear optical element can be used.

Examples of the temperature-regulating means include known heating means and cooling means, and combinations thereof. As the detection of the temperature, the internal temperature of the biopolymer solution, a mixed liquid etc. may be detected, or an ambient external temperature may be detected. The temperature-regulating means may be equipped with a program circuit for performing necessary temperature regulation.

The apparatus usable for the method for producing a crystal of the invention may be equipped, if necessary, with an apparatus, a circuit or a program for detecting the generation of a crystalline nucleus in the solution, pH of the solution etc. and for regulating these. For the detection and the control of crystallization conditions, an apparatus, wherein a plurality of cells for detecting crystallization conditions are made into one chip, is preferable. Such chip for detection can be produced by a general production process of a semiconductor apparatus, as described in JP-A-2001-213699.

An apparatus usable for the method for producing a crystal, in particular a biopolymer crystal, may also be equipped with such a means that uses a laser light of a long wavelength that is not absorbed by the biopolymer, for detecting the generation situation of the crystalline nucleus although it does not contribute to the generation of the crystalline nucleus or to the crystal growth.

The biopolymer crystal obtained by the method for producing a crystal of the invention is not only offered as a sample for X ray crystal structure analysis, but may also be used for medical compositions as a formulation for prevention or treatment, because generally the crystal has an extremely high storage stability, and, since the biopolymer is in a crystal form, particularly advantageous administration becomes possible. The biopolymer crystal is suitable for, for example, oral, subcutaneous, intracutaneous, intraperitoneal, intravenous or intramuscular administration, etc. The biopolymer crystal obtained by the method for producing a crystal of the invention can be preferably used for medical compositions consisting of a pharmacologically effective dose of the crystallized biopolymer as an active substance and, if necessary, one kind or two or more kinds of common pharmaceutically acceptable carriers.

The biopolymer crystal obtained by the method for producing a crystal of the invention can be used, for example, as a depot formulation for administrating a daily dosage of biopolymer 0.001 µg/kg bodyweight to 100 mg/kg bodyweight that is effective pharmacologically, in pharmaceutical formulations, theoretically by the same method as those known for many biopolymers. Accordingly, various biopolymers in a broad range can be used in the form of being crystallized by the invention, for example, as a therapeutic agent depot formulation, an antigen depot formulation, a DNA depot formulation or a sugar depot formulation. A crystallization auxiliary agent contained in the crystal is used, furthermore, as an adjuvant (in vaccination).

### Examples

Hereinafter, Examples of the present invention are shown, but the invention shall not be restricted by these Examples.

### (Example 1)

A cover glass (diameter: 22 mm, thickness: 0.2 mm) shown in Fig. 1 was prepared. In a region of 0.5 mm × 1.0 mm at the center of the cover glass, gold nano structure has been built. In the gold nano structure, as shown in Fig. 2, a cuboid structure of a height 30 nm, and a regular square of one side 100 nm are repeatedly built in a checkerboard pattern at an interval of 200 nm.

So as to cover the gold nano structure, 10 micro litters of a protein solution was dropped.

The composition of the protein solution used was composed of 15 mg/mL hen egg white lysozyme, and a 50 mM sodium acetate buffer solution of pH 4.3 containing 0.7 M sodium chloride.

The cover glass to which the protein solution had been dropped was placed in a batch plate (a 96-hole batch plate for vapor diffusion, DI-038, manufactured by HAMPTON RESEARCH CORP). In the batch plate, a reservoir solution containing sodium chloride of the same concentration as that of the protein solution was dropped on the periphery of the cover glass, and the batch plate was covered so that the evaporation of the protein solution does not occur.

For the light irradiation, light from a xenon lamp (a 300 W xenon lamp, manufactured by Ushio, Inc.) was used. Out of the irradiated light from the xenon lamp, light having passed through a water filter for absorbing infrared light, and a cut filter of 400 nm for absorbing ultraviolet light was irradiated. To the solution on the cover glass in the batch plate, the xenon lamp light was irradiated for 30 minutes, which was stood still in an incubator at 20°C for 7 days.

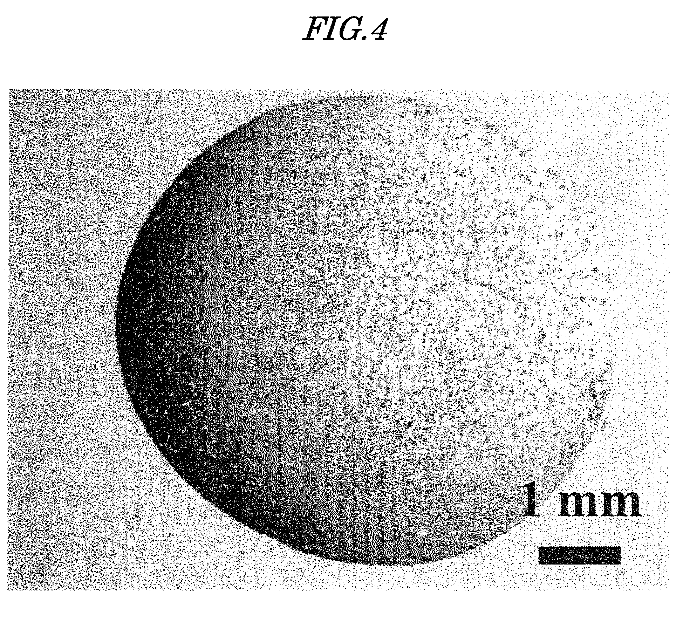

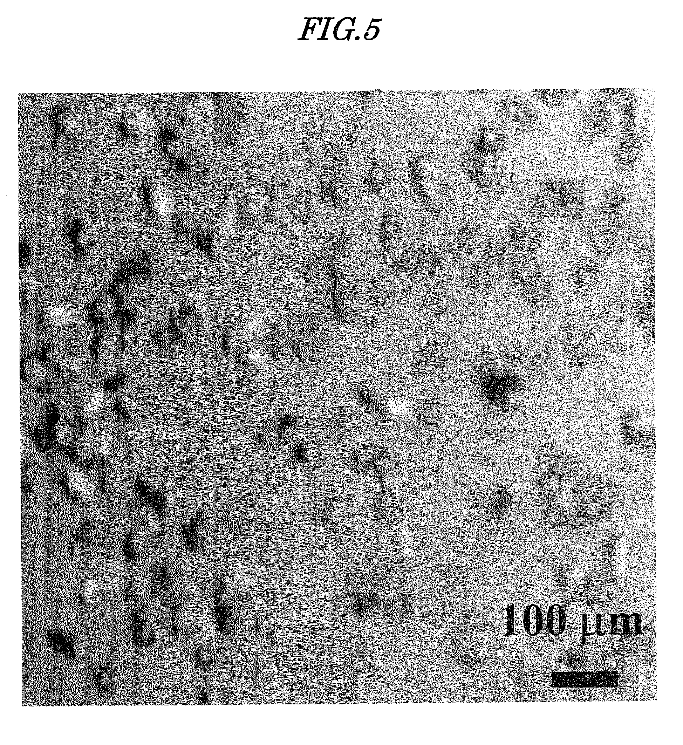

After the still standing, the number of protein crystals that appeared from the solution was about 4,000. The crystals were tetragonal crystals of hen egg white lysozyme. The result is shown in Fig. 4, and Fig. 5 that is a partially enlarged drawing of Fig. 4.

### (Comparative Example 1)

The procedure in Example 1 was repeated, except that the cover glass having the gold nano structure was replaced by a cover glass not having the gold nano structure and the light irradiation by a xenon lamp light was not performed.

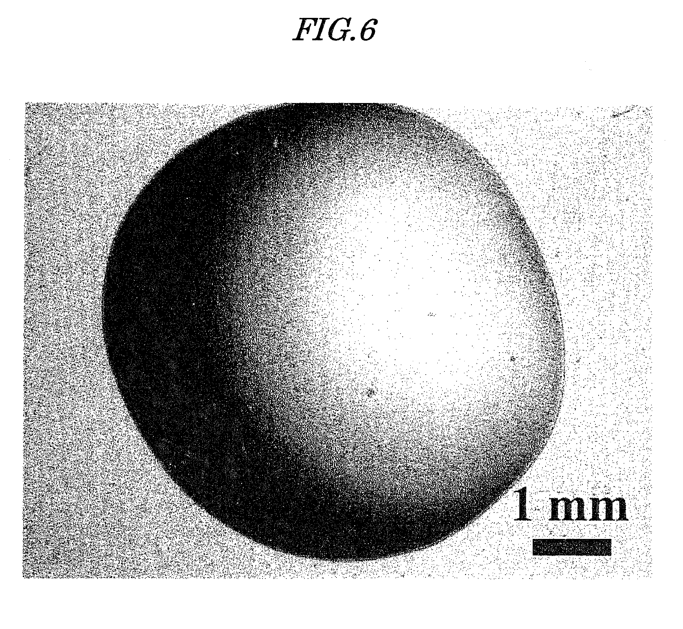

After the still standing, the number of the protein crystals that appeared from the solution was 1. The crystal was a tetragonal crystal of hen egg white lysozyme. The result is shown in Fig. 6.

### (Comparative Example 2)

The procedure in Example 1 was repeated, except that cover glass having the gold nano structure was replaced by a cover glass not having the gold nano structure.

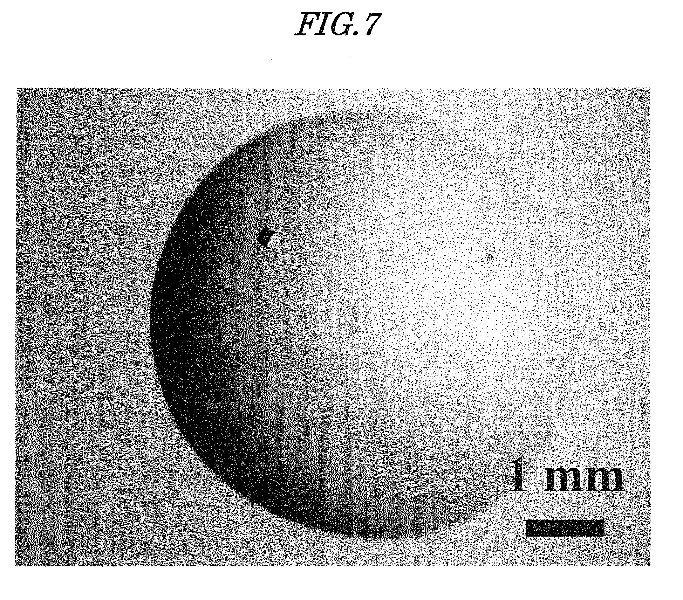

After the still standing, the number of the protein crystals that appeared from the solution was 1. The crystal was a tetragonal crystal of hen egg white lysozyme. The result is shown in Fig. 7.

### (Example 2)

The procedure in Example 1 was repeated, except that the light irradiation by a xenon lamp light was not performed.

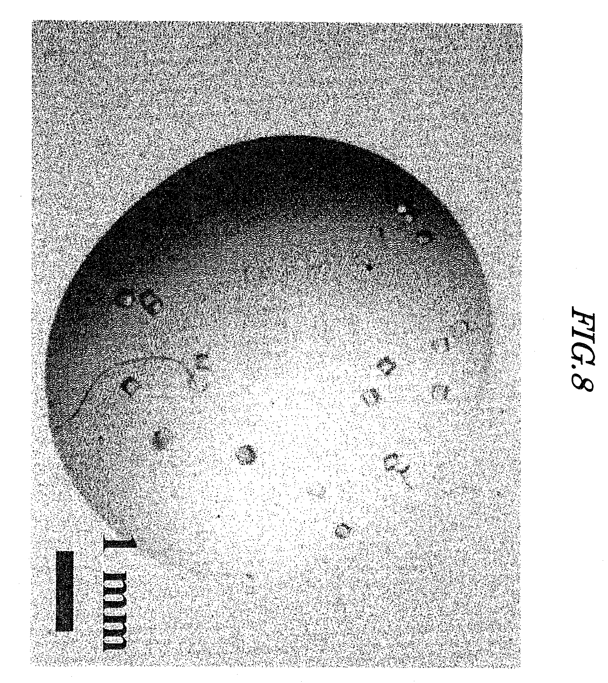

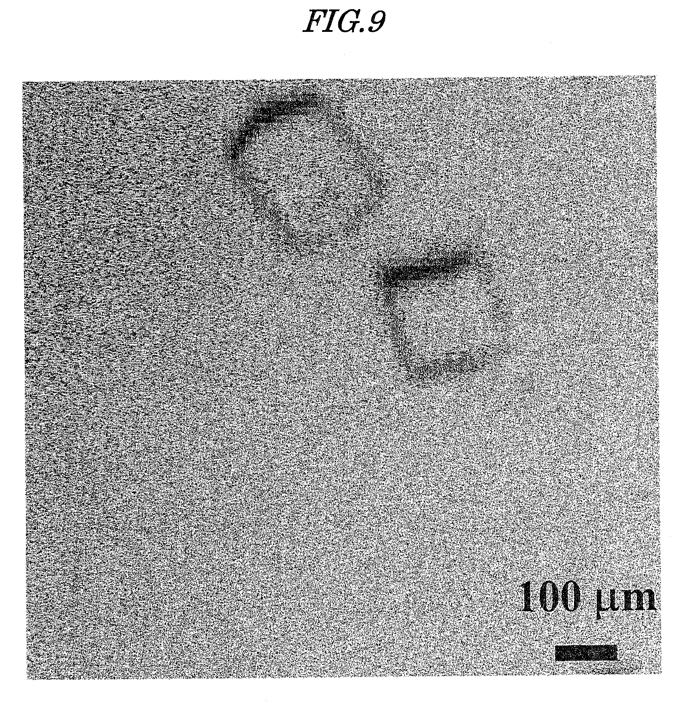

After the still standing, the number of the protein crystals that appeared from the solution was 20. The crystal was a tetragonal crystal of hen egg white lysozyme. The result is shown in Fig. 8, and Fig. 9 that is a partially enlarged drawing of Fig. 8.

### (Comparative Examples 3 and 4)

Two cover glasses, for which only one gold nano structure in a square shape of 100 nm on a side was arranged at the center, were prepared.

The shape of the cover glasses other than the gold nano structure is the same as that used in Example 1. Onto the nano structure, the protein solution was dropped, which was still-stood on the batch plate. To the batch plate, a reservoir solution was added, which was covered.

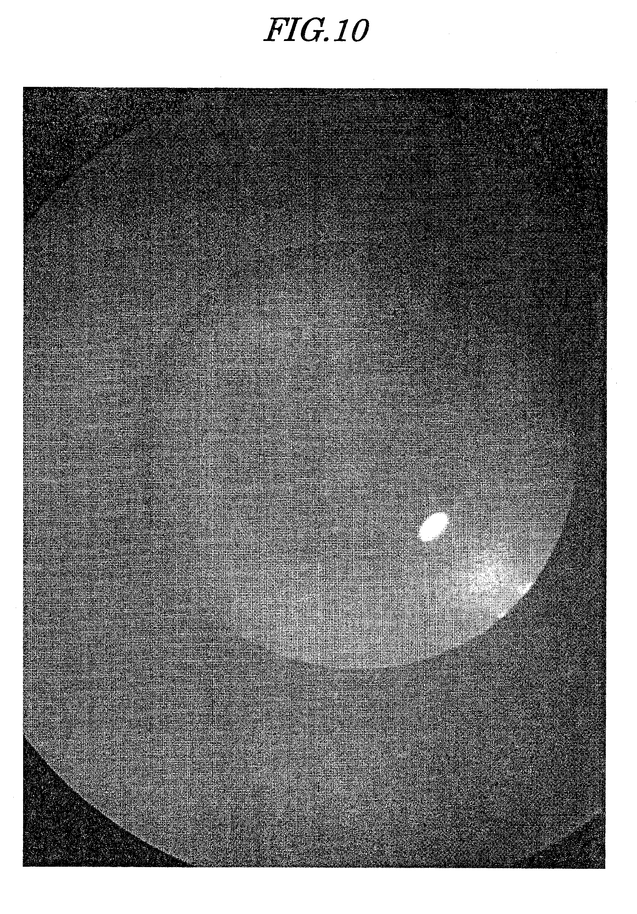

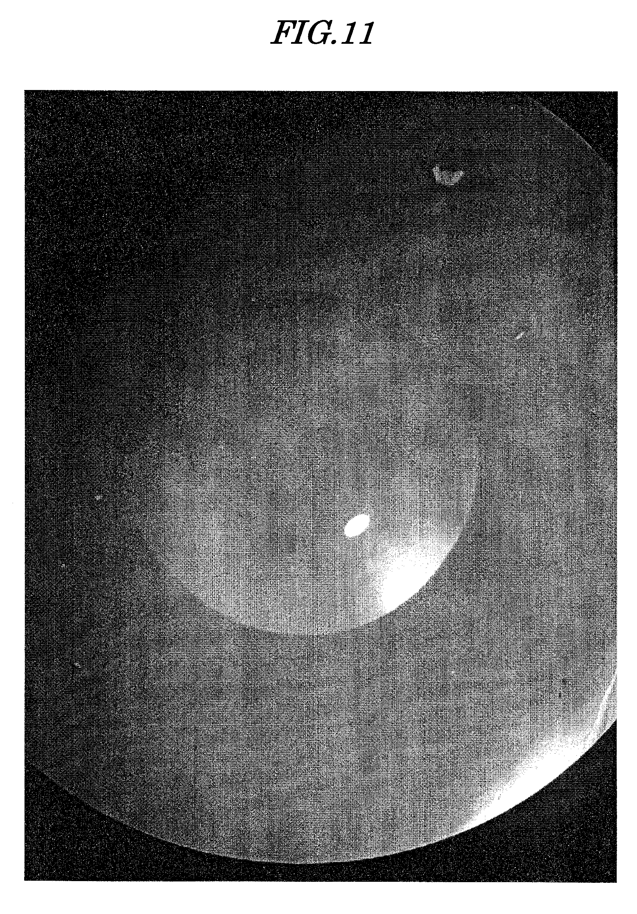

To one of the cover glasses, xenon lamp light was irradiated for 30 minutes in the same method as in Example 1 (Comparative Example 3). To the other cover glass, no light was irradiated as a reference sample (Comparative Example 4). When they were observed after several days, no crystal had appeared from either of the drops. Fig. 10 shows the result of Comparative Example 3 to which the xenon lamp light was irradiated, and Fig. 11 shows the result of Comparative Example 4 to which no xenon lamp light was irradiated.

## Claims

1. A container for crystallization of a biopolymer, comprising a structure wherein two or more noble metals and/or noble metal-coated bodies are arranged at an interval of 1 to 1,000 nm.

2. The container for crystallization of a biopolymer according to claim 1, wherein the noble metal is gold, silver, platinum, and/or an alloy thereof.

3. The container for crystallization of a biopolymer according to claim 1 or 2, wherein the noble metal is gold.

4. The container for crystallization of a biopolymer according to any one of claims 1 to 3, wherein at least two among the noble metals and/or the noble metal-coated bodies are arranged at an interval of 1 to 500 nm.

5. The container for crystallization of a biopolymer according to any one of claims 1 to 4, wherein the container has the structure that is directly provided on the surface of the container, or the container has a substrate for which the structure is provided in the container.

6. The container for crystallization of a biopolymer according to any one of claims 1 to 5, wherein length, width, and height of the noble metals and/or noble metal-coated bodies is each independently 5 to 500 nm.

7. A crystallization apparatus of a biopolymers, comprising the container for crystallization of a biopolymer according to any one of claims 1 to 6.

8. A method for producing a biopolymer crystal, comprising the steps of:
preparing the container for crystallization of a biopolymer according to any one of claims 1 to 6, and
making the structure contact with a biopolymer solution.

9. The method for producing a biopolymer crystal according to claim 8, further comprising a step of irradiating light to the structure contacting with the biopolymer solution.

10. The method for producing a biopolymer crystal according to claim 9, wherein the light is a light having a wavelength that is longer than 400 nm.

11. A substrate for crystallization of a biopolymer, having a structure wherein two or more noble metals and/or noble metal-coated bodies are arranged at an interval of 1 to 1,000 nm.
